# EUROPEAN PATENT SPECIFICATION

(11) **EP 1 767 536 B1**
(45) Date of publication and mention of the grant of the patent: **29.06.2011**
(21) Application number: 05741956.6
(22) Date of filing: 13.04.2005
(51) Int. Cl.: C07D 471/04, C07D 215/56, A61K 31/4709, A61K 9/00, A61P 31/04, C07D 221/00, C07D 209/00

(54) **QUINOLONECARBOXYLIC ACID COMPOUNDS, PREPARATION METHODS AND PHARMACEUTICAL USES THEREOF**
CHINOLINCARBONSÄUREDERIVATE, DEREN HERSTELLUNGSVERFAHREN UND PHARMAZEUTISCHE VERWENDUNG
DERIVES D'ACIDE CARBOXYLIQUE DE QUINOLONE, METHODES DE PREPARATION ET LEUR UTILISATION PHARMACEUTIQUE

(30) Priority: 21.04.2004 CN 200410033956
(43) Date of publication of application: 28.03.2007
(73) Proprietor: Institute of Medicinal Biotechnology, Chinese Academy of Medical Sciences, Beijing 100050 (CN); Xinchang Pharmaceutical Factory, Zhejiang Medicine Co. Ltd., Xinchang Zhejiang 312500 (CN)
(72) Inventor: GUO, Huiyuan, Chongwen District, Beijing 100050 (CN); LIU, Jiuyu, Chongwen District, Beijing 100050 (CN)
(74) Representative: Schüssler, Andrea
(86) International application number: PCT/CN2005/000489
(87) International publication number: WO 2005/103048

(56) References cited:
- EP-A- 0 550 903
- WO-A-01/45679
- CN-A- 1 074 218
- CN-A- 1 211 984
- CN-A- 1 245 428
- CN-A- 1 247 865
- CN-A- 1 427 815
- CN-A- 1 431 189
- YOSHIDA TOSHIHIKO ET AL: "Studies on Quinolone Antibacterials. IV. Structure-Activity Relationships of Antibacterial Activity and Side Effects for 5- or 8-Substituted and 5,8-Disubstituted-7-(3-amino-1-pyrrolidiny l)-1-cyclopropyl-1,4-dihydro-4-oxoquinolin e-3-carboxylic Acids" CHEMICAL AND PHARMACEUTICAL BULLETIN, vol. 44, no. 5, 1996, pages 1074-1085, XP002538936

## Description

### Field of the invention

The present invention relates to quinolonecarboxylic acid compounds, their salts and hydrates, preparation methods thereof, and antibacterial drug compositions containing the same.

### Background of the invention

Quinolones are broad-spectrum, high performance and low toxicity synthetic drugs. Early quinolones have potent activity against gram-negative bacteria, but inferior activity against gram-positive bacteria. Although newly available quinolones such as gatifloxacin (Drug, 1999, 58(4):683) and moxifloxacin (World Notes on Antibiotics, 2002, 23(6):274) exhibit improved antibacterial activity, the activity against gram-positive bacteria, and certain bacteria such as S. pneumoniae, enterococcus, etc., needs to be further strengthened. With introduction of difluoromethoxy group at C-8 position of quinolone core, T-3811, a novel quinolone with hydrogen atom at C-6 position of quinolone core (Chinese New Drugs Journal, 2002, 11(10):766), which entered phase III clinical trial in 2001, exhibits enhanced in vitro activity against S. pneumoniae, even superior to gatifloxacin.

WO 01/45679 A describes the use of chemotherapeutic agents for the production of a medicament for the topical and/or local treatment of diseases caused by bacteria and/or for prophylaxis in human or animals. According to WO 01/45679 A, chemotherapeutics are naphthyridon carboxylic acid compounds or derivatives of quinolonecarboxylic acid, such as moxifloxacin. Compared with WO 01/45679 A, the present invention provides a novel selection from WO 01/45679 A in view of the choice of the specific A rings, wherein comparison data show that the compounds of the present invention have a better anti-bacterial activity.

### Detailed description of the invention

Based on the aforementioned prior art, the present invention aims to provide novel quinolonecarboxylic acid compounds, their salts and hydrates.

The compounds, their salts and hydrates show potent in vitro antibacterial activity against broad-spectrum pathogenic bacteria, superior in vitro activity against S. pneumoniae compared to gatifloxacin, favorable pharmacokinetics, and low toxicity. The compounds, which possess a hydrogen atom or an amino group at C-5 position, cis-substituted optical or racemic 2,8-diazo-dicyclo[4,3,0]nonanyl at C-7 position, and difluoromethoxyl at C-8 position of quinolone core, have superior activity against gram-positive bacteria and broad spectrum antibacterial activity compared with the known quinolones.

The invention also provides preparation method for the quinolonecarboxylic acid compounds, their salts and hydrates, and antibacterial drug compositions containing the same.

As a solution to the above problem, the technical proposal of the invention is as follows: 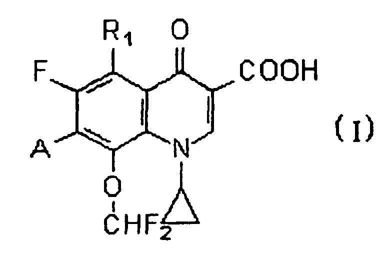 The present invention relates to quinolonecarboxylic acid compounds of formula (I), pharmaceutically acceptable salts or hydrates thereof,
wherein R₁ is a hydrogen atom or an amino group, A has structure of formula (II) with (1S, 6S) configuration, structure of formula (III) with (1R, 6R) configuration, or structure of formula (IV) with cis-racemic configuration, 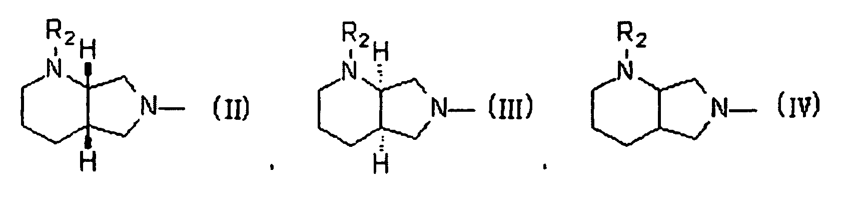 in which R₂ is a hydrogen atom or an amino-protecting group.

The amino-protecting group in this invention refers to formyl, acetyl, trifluoroacetyl, benzoyl, paranitrobenzoyl, p-toluene sulfonyl, methoxycarbonyl, ethoxycarbonyl, fluorenyl methoxycarbonyl, t-butyl-oxycarbonyl (BOC) or trichloroethoxycarbonyl.

The salts in this invention are ones of inorganic acids, organic acids or other acids generally known and conventionally applied to quinolone technical field. The salts of inorganic acids can be salt of hydrochloric acid, hydrobromic acid, phosphoric acid, or sulfuric acid. The salts of organic acids can be a salt of methanesulfonic acid, p-toluene sulfonic acid, acetic acid, trifluoroacetic acid, lactic acid, citric acid, maleic acid, oxalic acid, succinic acid, benzoic acid, tartaric acid, fumaric acid, mandelic acid, ascorbic acid, or malic acid.

The invention also provides method for preparing compound of formula (I) by reacting compound of formula (V) with compound of formula (VI), wherein R₁ is a hydrogen atom or an amino group, R₃ is an amino-protecting group, and X is a halogen atom.

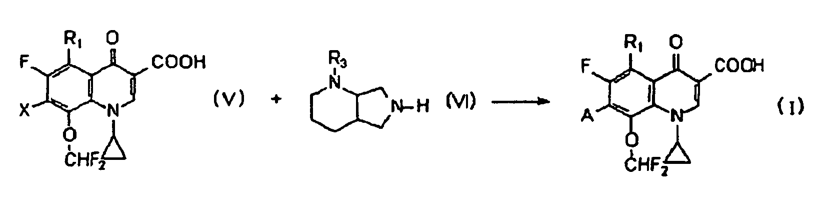

The detailed method comprises reacting compound of formula (V) with compound of formula (VI) in the presence of acid-binding agent under stirring at temperature within room temperature to 200°C for 1-20 h, and removing amino-protecting group of compound (I), wherein the amino-protecting group can be formyl, acetyl, trifluoroacetyl, benzoyl, paranitrobenzoyl, p-toluene sulfonyl, methoxycarbonyl, ethoxycarbonyl, fluorenyl methoxycarbonyl, t-butyl-oxycarbonyl (BOC) or trichloroethoxycarbonyl, and the halogen atom can be fluorine, chlorine or bromine.

The solvent for the aforementioned reaction can be any solvent having no adverse effect on the reaction, preferably pyridine, acetonitrile, N,N-dimethylformamide, dimethyl sulfoxide, N-methyl pyrrolidone or hexamethyl phosphoramide.

The reaction is commonly carried out in the presence of acid acceptor. In such a case, reactant (VI) is used in excess to increase the reaction efficiency of the relatively expensive starting material (V), for example, molar ratio of compound (VI) to compound (V) is 1-10, preferably 1-5, wherein after the reaction, unreacted compound (VI) can be recovered and reused for another reaction. The acid acceptor is preferably selected from inorganic base, such as sodium bicarbonate, or potassium carbonate, etc., and organic base, such as pyridine, triethyl amine, diisopropylethylamine, N,N-dimethylaniline, N,N-dimethyl aminopyridine, 1,8-diazabicyclo-[5.4.0]undecylene-7-ene or 1,4-diazabicyclo-[2.2.2]octane.

The amino-protecting group of compound (I) can be removed by hydrolysis or solvolysis according to relevant properties of the protecting group. For example, the protecting group can be removed by treating at 0-130°C in solvent and optionally in the presence of acid or base. The acid useful for this purpose includes inorganic acid, such as hydrochloric acid, hydrobromic acid, sulfuric acid, and phosphoric acid, etc.; organic acid, such as acetic acid, trifluoroacetic acid, formic acid, and toluenesulfonic acid, etc.; or Lewis acid, such as boric tribromide, and aluminum chloride, etc. The base useful for this purpose includes hydroxide of alkali metal or alkaline earth metal, such as sodium hydroxide, and barium hydroxide, etc.; carbonate of alkali metal, such as sodium carbonate, and calcium carbonate, etc.; alkoxide of alkali metal, such as sodium methoxide, and sodium ethoxide, etc. The reaction can be carried out in the presence of solvent, such as water and/or organic solvent including ethanol, tetrahydrofuran, dioxan, ethylene glycol, and acetic acid, etc. If desired, the reaction also can be carried out in absence of any solvent.

The present invention further provides another method for preparing compound of formula (I) by firstly converting compound (VII) into compound (VIII), and then reacting compound (VIII) with compound (VI) to obtain compound (I) via intermediate compound (IX), 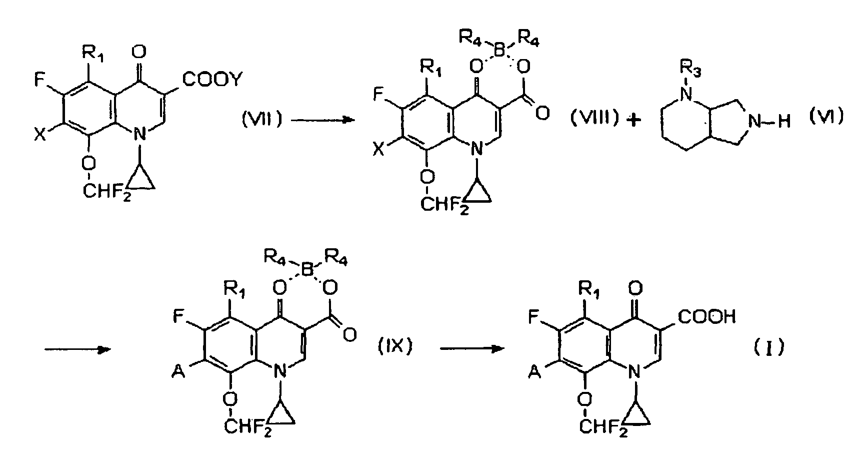 wherein R₁ is a hydrogen atom or an amino group, Y is hydrogen, C₁-C₆ alkyl, or optionally substituted benzyl, R₄ is C₂-C₆ aliphatic acid group, C₂-C₆ halogenated aliphatic acid group, or C₇-C₁₁ aromatic acid group.

And the formula (VI) concerning chiral compound represents three optical isomers possessing (1S,6S) configuration, (1R,6R) configuration, or cis- racemic configuration.

The method specifically comprises reacting compound (VIII) with compound (VI) under stirring in the presence of acid-binding agent for 1-20 h at temperature within room temperature to 200 °C to give compound (IX), and removing boron-containing group and amino-protecting group of the compound (IX).

In the aforementioned scheme, R₁, A, R₃, and X are as defined above, Y represents H, C₁-C₆ alkyl, and optionally substituted benzyl; R₄ represents C₂-C₆ aliphatic acid group, C₂-C₆ halogenated aliphatic acid group, or C₇-C₁₁ aromatic acid group. The formula (VI) concerning chiral compound represents three optical isomers possessing (1S,6S), or (1R,6R) configuration or cis-racemic configuration.

Method of converting compound (VII) into compound (VIII) is known, and can be easily realized according to already disclosed method (see EP 0352123). Compound (IX) can be prepared by reacting compound (VIII) with compound (VI) under stirring in the presence of solvent and appropriate amount of base at temperature within room temperature to 200°C for 1-20 h.

The solvent for the aforementioned reaction can be any solvent having no adverse effect on the reaction, preferably pyridine, acetonitrile, N,N-dimethylformamide, dimethyl sulfoxide, N-methyl pyrrolidone or hexamethyl phosphoramide.

The reaction is commonly carried out in the presence of acid acceptor, In such a case, reactant (VI) is used in excess to increase the reaction efficiency of the relatively expensive starting material (VIII), for example, molar ratio of compound (VI) to compound (VIII) is 1-10, preferably 1-5, wherein after the reaction, unreacted compound (VI) can be recovered and reused for another reaction. The acid acceptor in the reaction is preferably selected from inorganic base, such as sodium bicarbonate, or potassium carbonate, etc., and organic base, such as pyridine, triethyl amine, diisopropylethylamine, N,N-dimethylaniline, N,N-dimethyl aminopyridine, 1,8-diazabicyclo-[5.4.0]undecylene-7-ene or 1,4-diazabicyclo-[2.2.2]octane.

Borate ester moiety of compound (IX) is hydrolyzed while N-deprotection reaction proceeds, thereby generating compound (I).

In the reaction, the amino-protecting group can be removed by hydrolysis or solvolysis according to relevant properties of the protecting group. For example, the protecting group can be removed by treating at 0-130°C in solvent and optionally in the presence of acid or base. The acid useful for this purpose includes inorganic acid, such as hydrochloric acid, hydrobromic acid, sulfuric acid, and phosphoric acid, etc.; organic acid, such as acetic acid, trifluoroacetic acid, formic acid, and toluenesulfonic acid, etc.; or Lewis acid, such as boric tribromide, and aluminum chloride, etc. The base useful for this purpose includes hydroxide of alkali metal or alkaline earth metal, such as sodium hydroxide, and barium hydroxide, etc.; carbonate of alkali metal, such as sodium carbonate, and calcium carbonate, etc.; alkoxide of alkali metal, such as sodium methoxide, and sodium ethoxide, etc. The reaction can be carried out in presence of solvent, such as water and/or organic solvent including ethanol, tetrahydrofuran, dioxan, ethylene glycol, and acetic acid, etc. If desired, the reaction also can be carried out in absence of any solvent.

The application further shows a novel method for preparing compound (VII), as is shown in the following scheme 3, in which R₁, X and Y are as defined above.

Mixture of halogen acid and acetic acid is generally selected as the reacting agent for converting compound (X) into (XI), preferably mixture of hydrobromic acid 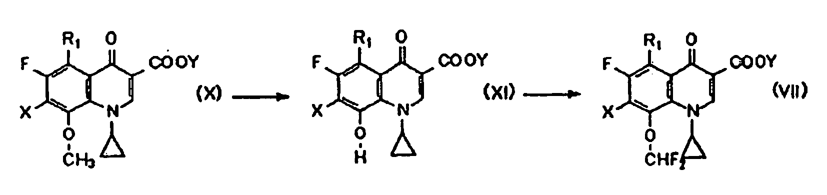 and acetic acid. The molar ratio of hydrobromic acid to compound (X) is 2-20, preferably 4-10, and volume ratio of acetic acid to hydrobromic acid is 1-10, preferably 1-4. The reaction is carried out at 0-120°C, preferably 90-110°C.

The reaction solvent for converting compound (XI) into (VII) can be selected from any solvent having no adverse effect on the reaction, preferably N,N-dimethylformamide, dimethyl sulfoxide, N-methyl pyrrolidone or hexamethyl phosphoramide.

The reaction is generally carried out by reacting compound (XI) with monochlorodifluoromethane in alkaline aqueous solution. The base useful for this purpose includes hydroxide of alkali metal or alkaline earth metal, such as sodium hydroxide, and barium hydroxide, etc.; carbonate of alkaline metal, such as sodium carbonate, and calcium carbonate, etc.; alkoxide of alkaline metal, such as sodium methoxide, and sodium ethoxide, etc.; wherein molar ratio of the base to compound (IX) is 1-5, preferably 2; and molar ratio of monochlorodifluoromethane to the base is 1-50, preferable 10-15.

Starting compound (X) and intermediate compound (XI) in reaction scheme 3 are known compounds (see CN87100580 for compound (X), see Chinese Journal of Pharmaceuticals for compound (XI), 2001, 36 (6): 419-422), but interconversion of the compound (X), (XI) and (V) has not been seen in any publication.

By method shown in reaction scheme 4, another starting compound (VI) can be obtained, wherein R₃ is as defined above, and R₅ represents amino-protecting group, such as optionally substituted benzyl group.

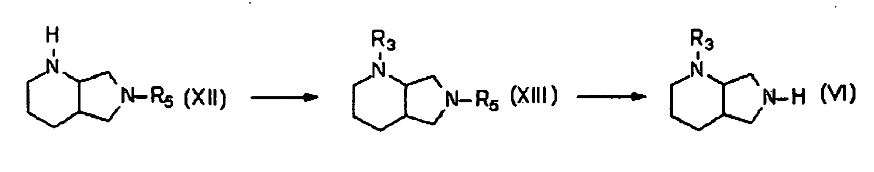

And compounds of formula (XII), (XIII), and (VI) concerning chiral compounds all represent optical isomers possessing (1S,6S), (1R,6R) configuration or cis-racemic configuration.

The aim of converting compound (XII) into compound (XIII) is to introduce amino-protecting group. The protecting group useful for this purpose includes formyl, acetyl, trifluoroacetyl, benzoyl, paranitrobenzoyl, p-toluene sulfonyl, methoxycarbonyl, ethoxycarbonyl, fluorenyl methoxycarbonyl, t-butyl-oxycarbonyl (BOC) or trichloroethoxycarbonyl, etc.

The aim of converting compound (XIII) into compound (VI) is to remove the various benzyl type protecting groups, which can be effectively removed by reduction. Although the reduction conditions for removing the protecting groups vary with the relevant properties of the protecting groups, the reduction is generally carried out in the presence of a catalyst (e.g. Pt, Pa, Raney Ni) in an inert solvent with hydrogen flow at 10-100°C, or with sodium metal or lithium metal and in ammonia water at -50°C to -10°C, preferably in the presence of 10% Pd/C under 5atm hydrogen atmosphere at room temperature.

The compound (XII) used as starting material in the reaction scheme 4 is known compound, and can be easily prepared according to the patent: CN 93100215.X

A corresponding acid is used in N-deprotection of compound (I) to give corresponding salt.

If desired, the salts of compound (I) can be further dissolved in water or other water-containing organic solvent to form a solution, to which an alkaline aqueous solution or alkaline aqueous organic solvent is added to adjust pH to 6-8, and cooled to separate out hydrate of the compound (I). The base useful for this purpose includes hydroxide of alkali metal or alkaline earth metal, such as sodium hydroxide, and barium hydroxide, etc.; carbonate of alkali metal, such as sodium carbonate, and calcium carbonate, etc.; alkoxide of alkali metal, such as sodium methoxide, and sodium ethoxide, etc.

The present invention also provides drug compositions which contain physiologically effective amount of quinolonecarboxylic acid derivatives 0.1-99.9 wt% and/or pharmaceutically acceptable carrier 0.1-99.9 wt%. The drug compositions exist in the form of pharmaceutically appropriate preparation including tablet, sugar-coated tablet, film-coated tablet, enteric coating tablet, slow release tablet, capsule, hard capsule, soft capsule, slow release capsule, oral liquid, mistura, buccal tablet, granule, pill, pulvis, concentrated extract, bolus, suspensoid, liquor, injection, powder and injection preparation, freeze drying powder and injection preparation, suppositorium, ointment, plaster, cream, spray, aerosol, gutt, and patch.

In a preparation form, the drug compositions in the present invention contain the inventive compound 0.1-1,000 mg per dose as effective quantity, wherein the "dose" means a preparation unit such as a tablet or a capsule, or the administration dosage for each time, for example, 100 mg per time.

When the drug compositions in the present invention are prepared into solid or semisolid pharmaceutical preparation in the form of pulvis, tablet, dispersible pulvis, capsule, cachet, suppositorium or ointment, a solid carrier can be used. The solid carrier useful for this purpose is preferably chosen from one or more of diluent, flavoring agent, solubilizer, lubricant, suspension, binder, and swelling agent; or it can be coating material. In a powder preparation, 5% or 10-70% micronized active ingredient is contained in the carrier. The suitable solid carrier can be selected from one or more magnesium carbonate, magnesium stearate, talcum, sucrose, lactose, pectin, dextrin, starch, gelatin, tragacanth, methyl cellulose, sodium carboxymethylcellulose, low boiling point wax, cocoa butter, etc. Tablet, powder, cachet and capsule are the most favorable preparations for oral administration because they are easy to administrate.

A liquid preparation according to the present invention includes solution, suspension, and emulsion. For example, an injectable preparation for parenteral administration can be in the form of water or water-propylene glycol solution, whose isotonic concentration, pH, etc. are adjusted to fit in vivo physiological conditions. The liquid preparation may also be made into the form of polyethylene glycol or water solution. Aqueous oral liquid can be prepared by dissolving the active ingredient in water, and adding appropriate colorant, flavoring agent, stabilizing agent and thickening agent. Micronized active ingredient can be dispersed in a viscous material, such as natural or synthetic gelatin, methyl cellulose, sodium carboxymethylcellulose or other known suspension to prepare aqueous suspension suitable for oral administration.

For the sake of easy administration and dosage equality, the aforementioned pharmaceutical preparation is preferably formulated into unit dosage form. The "unit dosage form" means the physical separation unit suitable for single dose, and each unit contains the active ingredient in precalculated quantity for realizing anticipated treatment effect. The unit dosage form can be in packaged form, such as tablet, capsule, or powder in a tube or vial, or ointment, gel or cream in a tube or vial.

Although the quantity of the active ingredient contained in each unit dosage form may vary, it is generally adjusted to the range of 1-500 mg according to the efficacy of the selected active ingredient.

When compound of formula (I) in the present invention is used as a drug for treating bacterial infection, the dosage is preferably 6-14 mg/kg body-weight in the first stage. But the dosage may also vary with the need of the patient, seriousness of the infection to be treated, and the compound selected.

Professionals in this field can determine the preferable dosage suitable for a certain situation according to conventional method. Generally, the initial dosage is lower than the optimal dosage of the active ingredient for the treatment, and then the dosage is gradually increased till the optimal treatment effect is realized. For the sake of conveniency, total daily dosage can be divided into several doses for administration.

As is described above, the present compound shows stronger antibacterial activity and broader antibacterial spectrum against various kinds of pathogenic bacteria including gram-negative bacteria and gram-positive bacteria. Compared with the known quinolone antibacterial drugs, such as moxifloxacin, the present compound exhibits stronger antibacterial activity to gram-negative bacteria. Particularly, the activity against gram-positive bacteria of the present compound is much stronger than that of any known quinolones.

The antibacterial activity of the present quinolonecarboxylic acid derivates, in comparison with that of moxifloxacin (of our own make, see Chinese Journal of Pharmaceuticlas, 2004, 35(3):129) and ciprofloxacin (provided by Tianjin Central Pharmaceutical Factory, Lot Number 990307) which have been widely used and hold a large market share, is shown in Table 1. The pharmaceutically acceptable salts or hydrates thereof have the same antibacterial activity.

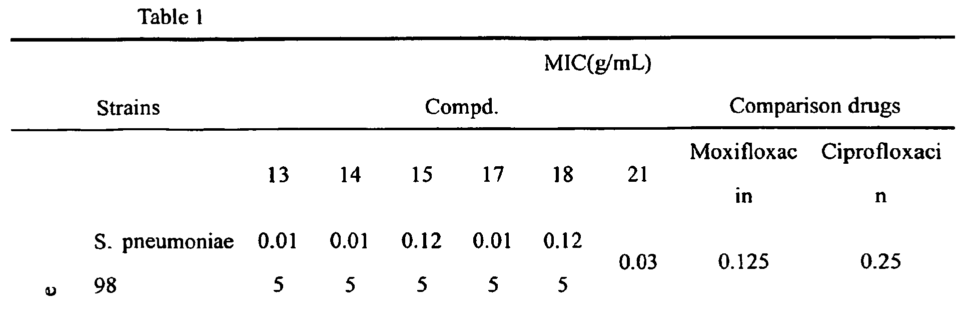

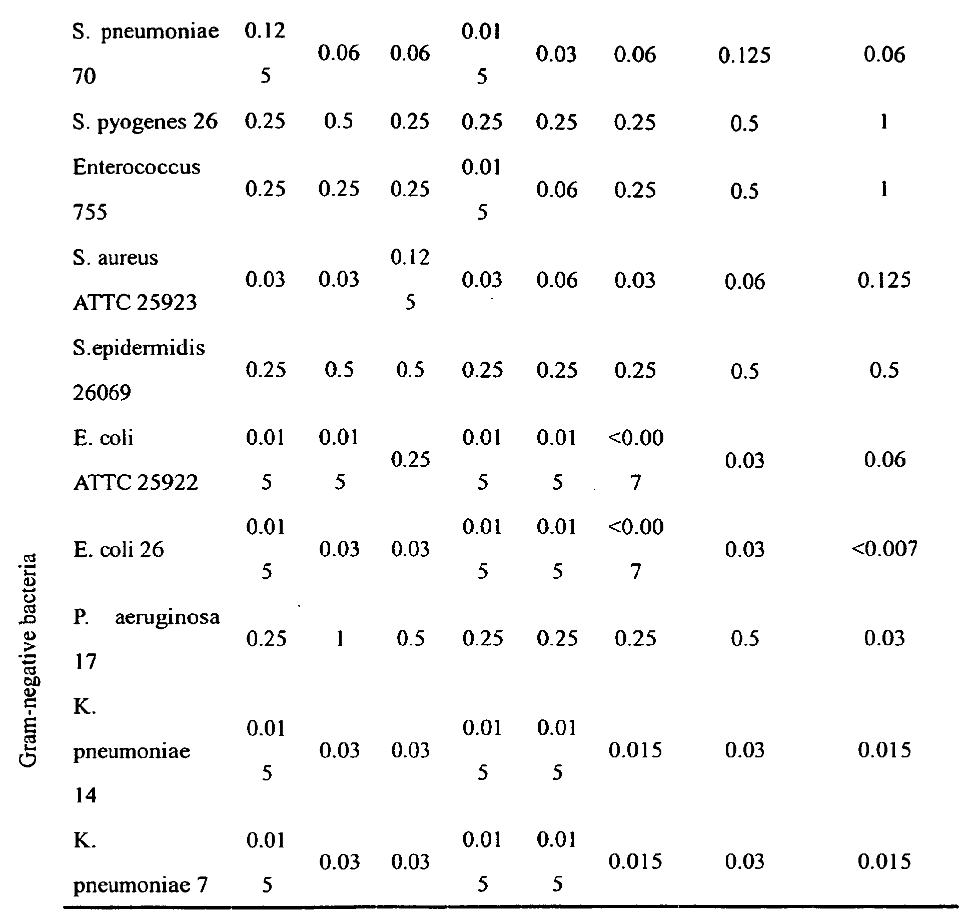

Two-fold plate dilution method is used for determination of activity, with Muener-Hinton medium (DIFCO) as the culture medium and clinically isolated pathogenic strain, quality control strain and standard strain (provided by National Institute for the Control of Pharmaceutical and Biological Products) as test strains.

With respect to pharmacokinetic performance, compared with known quinolones, the present compounds possess proper water solubility so that they can be well absorbed in vivo, exhibit extraordinarily high bioavailability, and are suitable as antibacterial agents.

In addition, due to their low toxicity, the present compounds can be effectively used in the prevention and treatment of diseases caused by bacterial infections in homoiotherm including human being.

### Acute Oral Toxicity Studies

Toxicity experiments are carried out on the compounds prepared in embodiment 11, 13 and 15 to determine the acute oral toxicity of the present compounds. Solutions of the three compounds in different concentrations are administered to male mice at a dosage of 0.1 mL/10g body weight, then the number of dead mice after 7 days is recorded, and lethal dose 50% (LD50) is calculated for each compound by Bliss program. The results are summarized in Table 3.

**Table 3: Acute oral toxicity of the test compounds in mice**

| Test Compound | LD50/(mg/Kg) |
|---|---|
| Embodiment 13 | >4000 |
| Embodiment 15 | >4000 |
| Embodiment 17 | >4000 |

The results of the experiments show that the three compounds possess low toxicity and are well suited for pharmaceutical uses.

### Embodiments

The following embodiments explain the present invention in further detail.

### Referential Example I

### Preparation of

### 5-Amino-1-cyclopropyl-6,7-difluoro-8-difluoromethoxyl-1,4-dihydro-4-oxoquinoli ne-3-carboxylic acid

The preparation comprises refluxing mixture of reducing iron powder (2.4 g, 42.8 mmol), water (4.5 mL) and acetic acid (0.3 mL) for 15 min under stirring, adding batch-wise ethyl 1-cyclopropyl-6,7-difluoro-8-difluoromethoxyl-5-nitro-1,4-dihydro-4-oxoquinoline-3-carboxylate (4.3 g, 10.6 mmol), adding ethanol (20 mL), continuously refluxing for 6 h under stirring, hot filtering, distilling off the solvent from the filtrate, adding concentrated hydrochloric acid (5 mL) and acetic acid (10 mL) to react at 100°C for 1 h, cooling to room temperature, filtering, adding ethanol (10 mL) to the filter cake to reflux for 0.5 h, cooling to room temperature, filtering, and drying obtained filter cake to give a pale yellow solid product 5-amino-1-cyclopropyl-6,7-difluoro-8-difluoromethoxyl-1,4-dihydro-4-oxoquinoli ne-3-carboxylic acid (3.1 g, 84.5%).
¹H NMR(DMSO), δ = 0.85-1.21(4H, m), 3.89-3.93(1H, m), 7.05(1H, t, J = 73.0Hz), 7.92(2H, br.), 8.62(1H, s), 14.25(1H, s). MS(m/z): 347(M⁺+1).

### Embodiment 1

### Preparation of 1-Cyclopropyl-6,7-difluoro-8-hydroxyl-1,4-dihydro-4-oxoquinoline-3-carboxylic acid

The preparation comprises dissolving Ethyl 1-cyclopropyl-6,7-difluoro-8-methoxy-1,4-dihydro-4-oxoquinoline-3-carboxylate (10.0g, 30.9mmol) in 40% HBr aqueous solution (20 mL) and acetic acid (20 mL) to react at 100°C, charging reaction mixture into ice water (500 mL) under fast stirring after the reaction finishes, going on with the stirring for 0.5 h, filtering, and drying obtained filter cake to give 1-cyclopropyl-6,7-difluoro-8-hydroxy-1,4-dihydro-oxoquinoline-3-carboxylic acid (7.8 g, 89.6%).
¹H NMR(DMSO) δ =1.01-1.27(4H, m), 4.25-4.33(1H, m), 7.67(1H, dd, J=8.1Hz, J=12.6Hz), 8.68(1H, s), 11.64(1H, s), 14.71(1H, br.). MS(m/z): 282(M⁺ +1).

### Embodiment 2

### Preparation of Ethyl 1-cyclopropyl-6,7-difluoro-8-hydroxyl-1,4-dihydro-4-oxoquinoline-3-carboxylate

The preparation comprises refluxing a mixture of 1-cyclopropyl-6,7-difluoro-8-hydrohyl-4-oxoquinoline- 3-carboxylic acid (target product of embodiment 1, 20.0 g, 71.2 mmol), concentrated sulfuric acid (5 mL) and ethanol (300 mL) to give a clear solution, cooling down to room temperature, filtering, washing obtained filter cake with ethanol, and drying to give 1-cyclopropyl-6,7-difluoro-8-hydroxy-1,4-dihydro-4-oxoquinoline-3-carboxylate (20.9 g, 95.0%).
¹H NMR(CDCl₃) δ =0.85-1.11(4H, m), 1.42(3H, t, J=6.9Hz), 4.33-4.40(1H, m), 4.42(2H, q, J=6.9Hz), 7.80-7.85(1H, m), 8.68(1H, s). MS(m/z): 310(M⁺ +1)

### Embodiment 3

### Preparation of Ethyl 1-cyclopropyl-6,7-difluoro-8-difluoromethoxyl-1,4-dihydro-4-oxo quinoline-3-carboxylate

The preparation comprises mixing 1-cyclopropyl-6,7-difluoro-8-hydroxyl-1,4-dihydro-4- oxoquinoline-3-carboxylate (target product of embodiment 2, 5.0 g, 16.2 mmol), NaOH (1.3 g, 32.4 mmol), and water (0.5 mL) with chlorodifluoromethane (25.0 g) dissolved in N,N-dimethyl formamide (100 mL) in a 250 mL autoclave, reacting for 6 h at 100 °C, cooling to room temperature, charging the reaction mixture into water (500 mL), stirring, filtering, and drying obtained filter cake to give ethyl 1-cyclopropyl-6,7-difluoro-8-difluoromethoxyl-1,4-dihydro-4-oxoquinoline-3-carb oxylate (3.8 g, 65.4%).
¹H NMR(CDCl₃) δ = 1.03-1.30(4H, m), 1.39(3H, t, J=6.9Hz), 3.95-4.00(1H, m), 4.38(2H, q, J=6.9Hz), 6.97(1H, t, J=72.9Hz), 8.21(1H, t, J=9.6Hz), 8.62(1H, s). MS(m/z): 360(M⁺ +1).

### Embodiment 4

### Preparation of ethyl 1-cyclopropyl-6,7-difluoro-8-difluoromethoxyl-5-nitro-1,4-dihydro-4-oxoquinolin e-3-carboxylate

The preparation comprises dissolving ethyl 1-cyclopropyl-6,7-difluoro-8-difluoromethoxyl-1,4-dihydro-4-oxoquinoline-3-carboxylate (target product of embodiment 3, 1.0 g, 2.8 mmol) in concentrated sulfuric acid (10 mL), slowly adding potassium nitrate (2.0 g, 19.8 mmol), reacting for 1 h at 40°C, cooling down to room temperature, charging the reaction mixture into ice water (100 mL), stirring, filtering, treating obtained filter cake with ethanol (20 mL), refluxing for 0.5 h, cooling down to room temperature, filtering, and drying obtained filter cake to give ethyl 1-cyclopropyl-6,7-difluoro-8-difluoromethoxyl-5-nitro-1,4-dihydro-4-oxoquinolin e-3-carboxylate (0.5 g, 45.5%).
¹H NMR(DMSO) δ =0.99-1.11(4H, m), 1.22(3H, t, J=7.0Hz), 3.88-3.92(1H, m), 4.18(2H, q, J=7.0Hz), 7.24(1H, t, J=72.0Hz), 8.56(1H, s). MS(m/z): 405(M⁺ +1).

### Embodiment 5

### Preparation of [1S,6S]-8-benzyl-2-tertbutyloxycarbonyl-2,8-diaza bicyclo[4,3,0]nonane

The preparation comprises stirring [1S,6S]-8-benzyl-2,8-diaza bicyclo[4,3,0] nonane(5.0 g, 23.1 mmol) in methanol (50 mL) at room temperature, adding batchwise tertbutyloxycarbonyl dicarbonate (5.1 g, 23.4 mmol), stirring for 0.5 h, vacuum distilling to remove solvent, and column chromatographing with petroleum ether and ethyl acetate at the ratio of 10:1 to give [1S,6S]-8-benzyl-2-tertbutyloxycarbonyl-2,8-diazabicyclo[4,3,0]nonane (7.1g, 97.2%),[α]²⁰D=-72.4° (CHCl₃,C=5).
¹H NMR(CDCl₃) δ =1.50(9H, s), 1.56-1.73(4H, m), 2.12(1H, br.), 2.53-2.78(5H, m), 3.62-3.72(2H, m), 3.86(1H, br.), 4.56(1H, br.), 7.23-7.32(5H, m). MS(m/z): 317(M⁺ +1).

### Embodiment 6

### Preparation of [1R,6R]-8-benzyl-2-tertbutyloxycarbonyl-2,8-diazabicyclo[4,3,0]nonane

As is in embodiment 5, [1R,6R]-8-benzyl-2-tertbutyloxycarbonyl-2,8-diazabicyclo[4,3,0]nonane is obtained by reacting [1R,6R]-8-benzyl-2,8-diaza bicyclo[4,3,0] nonane with tertbutyloxycarbonyl dicarbonate, [α]²⁰D=+71.8° (CHCl₃,C=5).
¹H NMR(CDCl₃) δ =1.50(9H, s), 1.57-1.73(4H, m), 2.14(1H, br.), 2.54-2.78(5H, m), 3.62-3.73(2H, m), 3.86(1H, br.), 4.57(1H, br.), 7.23-7.35(5H, m). MS(m/z): 317(M⁺+1).

### Embodiment 7

### Preparation of cis-8-benzyl-2-tertbutyloxycarbonyl-2,8-diaza bicyclo[4,3,0] nonane

As is in embodiment 5, cis-8-benzyl-2-tertbutyloxycarbonyl-2,8-diazabicyclo[4,3,0]nonane is obtained by reacting cis-8-benzyl-2,8-diazabicyclo[4,3,0]nonane with tertbutyloxycarbonyl dicarbonate.
¹H NMR(CDCl₃) δ 1.50(9H, s), 1.55-1.73(4H, m), 2.12(1H, br.), 2.53-2.79(5H, m), 3.65-3.74(2H, m), 3.84(1H, br.), 4.55(1H, br.), 7.23-7.35(5H, m). MS(m/z): 317(M⁺ +1).

### Embodiment 8

### Preparation of cis-8-benzyl-2-acetyl-2,8-diazabicyclo[4,3,0] nonane

The preparation comprises dissolving cis-8-benzyl-2,8-diazabicyclo[4,3,0]nonane (5.0 g, 23.1 mmol) in dichloromethane (50 mL) and triethylamine (5 mL) under stirring in ice bath, dropwise adding a solution of acetyl chloride (1.8 g, 23.1 mmol) in chloroform (10 mL), continuing with the stirring until the reaction finishes, distilling off the solvent, and chromatographing on silica gel with petroleum ether and ethyl acetate to give cis-8-benzyl-2-acetyl-2,8-diazabicyclo [4,3,0] nonane(5.1 g, 85.5%).
¹H NMR(CDCl₃) δ 1.68-1.85(4H, m), 1.98(3H, s), 2.21(1H, br.), 2.58-2.87(5H, m), 3.68-3.73(2H, m), 3.87(1H, br.), 4.58(1H, br.), 7.23-7.36(5H, m). MS(m/z): 259(M⁺+1).

### Embodiment 9

### Preparation of [1S,6S]-2-tertbutyloxycarbonyl-2,8-diaza bicyclo[4,3,0] nonane

The preparation comprises dissolving [1S,6S]-8-benzyl-2-tertbutyloxycarbonyl-2,8-diazabicyclo[4,3,0] nonane (target product of Embodiment 5, 5.0 g, 15.8 mmol) in methanol (50 mL), adding 10% Pd/C (1.0 g) as catalyst for hydrogenation under 3 atm for 10 h, filtering to remove the catalyst, concentrating the filtrate, adding petroleum ether to solidify the product, filtering, and drying obtained filter cake to give [1S,6S]-2-tertbutyloxycarbonyl-2,8-diaza bicyclo[4,3,0] nonane(3.1 g,86.8%), [α]²⁰ D = -70.2° (C=4).
¹H NMR(CDCl₃) δ =1.45(9H, s), 1.66-1.70(2H, m), 2.03-2.11 (2H, m), 2.72-2.88(2H, m), 3.11-3.78(5H, m), 3.92-3.97(1H, m), 4.53-4.55(1H, m). MS(m/z): 227(M⁺ +1).

### Embodiment 10

### Preparation of [1R,6R]-2-tertbutyloxycarbonyl-2,8-diaza bicyclo[4,3,0] nonane

[1R,6R]-8-benzyl-2-tertbutyloxycarbonyl-2,8-diazabicyclo[4,3,0]nonane (target product of embodiment 6) is catalytically hydrogenated according to the procedure similar to embodiment 9 to give [1R,6R]-2-tertbutyloxycarbonyl-2,8-diaza bicyclo[4,3,0]nonane, [α]²⁰D=+84.1° (C=4).
¹H NMR(CDCl₃) δ =1.45(9H, s), 1.66-1.71(2H, m), 2.03-2.11(2H, m), 2.71-2.89(2H, m), 3.10-3.77(5H, m), 3.92-3.99 (1H, m), 4.53-4.56(1H, m). MS(m/z): 227(M⁺ +1).

### Embodiment 11

### Preparation of cis-2-tertbutyloxycarbonyl-2,8-diazabicyclo[4,3,0]nonane

cis-8-Benzyl-2-tertbutyloxycarbonyl-2,8-diazabicyclo[4,3,0]nonane (target product of embodiment 7) is catalytically hydrogenated according to the procedure similar to embodiment 9 to give cis-2-tertbutyloxycarbonyl-2,8-diazabicyclo[4,3,0] nonane.
¹H NMR(CDCl₃) δ =1.45(9H, s), 1.66-1.71(2H, m), 2.03-2.13(2H, m), 2.70-2.87(2H, m), 3.11-3.79(5H, m), 3.92-3.99 (1H, m), 4.50-4.55(1H, m). MS(m/z):227(M⁺ +1).

### Embodiment 12

### Preparation of cis-2-acetyl-2,8-diazabicyclo[4,3,0]nonane

cis-8-Benzyl-2-acetyl-2,8-diazabicyclo[4,3,0]nonane (embodiment 8 compound) is catalytically hydrogenated according to the procedure similar to embodiment 9 to give cis-2-acetyl-2,8-diazabicyclo[4,3,0]nonane.
¹H NMR(CDCl₃) δ =1.64-1.70(2H, m), 2.01(3H, s), 2.01-2.11(2H, m), 2.70-2.88(2H, m), 3.08-3.78(5H, m), 3.90-3.99 (1H, m), 4.50-4.57(1H, m). MS(m/z): 169(M⁺ +1).

### Embodiment 13

### Preparation of 1-cyclopropyl-8-difluoromethoxyl-7-[(1S,6S)-2,8-diazabicyclo[4,3,0]nonane-8-yl] -6-fluoro-1,4-dihydro-4-oxoquinoline-3-carboxylic acid hydrochloride

The preparation comprises reacting boric acid (1.1 g, 17.8 mmol) with acetic anhydride (6 mL, 62.5 mmol) at 110°C for 1.5 h, cooling, adding glacial acetic acid (7.1 mL) to further react at 110°C for 1 h, naturally cooling to 50-60°C, adding ethyl 1-cyclopropyl-6,7-difluoro-8-difluoromethoxyl-1,4-dihydro-4-oxoquinoline-3-carb oxylate (target product embodiment 3, 4.3 g, 12.0 mmol), stirring for 6 h, vacuum distilling to remove solvent, charging into ice water (150 mL), stirring for 0.5 h, filtering, washing obtained filter cake with water and ethanol in turn, drying to give a white solid (5.1 g), reacting the obtained white solid (0.5 g, 1.1 mmol) with [1S,6S]-2-tertbutyloxycarbonyl-2,8-diaza bicyclo[4,3,0]nonane (target product of embodiment 9, 0.7 g, 3.1 mmol) in pyridine (20 mL) as solvent at room temperature for 2 h under stirring, distilling off the solvent to give a residue (0.6 g), adding acetyl chloride (5 mL) slowly into methanol (10 mL) under ice-cooling, stirring for 30 min at room temperature to prepare a solution of methanol/methyl acetate in anhydrous HCl, adding the aforementioned residue (0.6 g) dissolved in methanol (5 mL), stirring for 0.5 h at room temperature, distilling to remove solvent, adding ethylether (50 mL) to solidify the product, filtering, and subjecting obtained filter cake to recrystallization with ethanol to give 1-cyclopropyl-8-difluoromethoxyl-7-[(1S,6S)-2,8-diazabicyclo[4,3,0] nonane-8-yl] -6-fluoro-1,4-dihydro-4-oxoquinoline-3-carboxylic acid hydrochloride(0.4g), [ α ]²²D=-172.5° , (C=1). Calculated result (measured value) of element analysis for the product: C 53.23 (53.31), H 4.89 (4.78), N 8.87 (9.01), Cl 7.48 (7.49).
¹H NMR(CF₃COOD) δ =1.10-1.53(4H, m), 1.92-2.01(4H, m), 2.93(1H, br.), 3.21(1H, br.), 3.59-3.62(1H, m), 3.98-4.40(6H, m), 6.47(1H, t, J = 72.0Hz), 8.04(1H, d, J = 13.5Hz), 9.24(1H, s). MS(m/z):438(M⁺ +1).

The following salts can be likewise prepared, e.g. 1-cyclopropyl-8-difluoromethoxyl-7-[(1S,6S)-2,8-diazabicyclo[4,3,0]nonane-8-yl] -6-fluoro-1,4-dihydro-4-oxoquinoline-3-carboxylic acid hydrobromate 1-cyclopropyl-8-difluoromethoxyl-7-[(1S,6S)-2,8-diazabicyclo[4,3,0]nonane-8-yl] -6-fluoro-1,4-dihydro-4-oxoquinoline-3-carboxylic acid sulfate

### Embodiment 14

### 1-cyclopropyl-8-difluoromethoxyl-7-[(1R,6R)-2,8-diazabicyclo[4,3,0]nonane-8-yl]

-6-fluoro-1,4-dihydro-4-oxoquinoline-3-carboxylic acid hydrochloride As is in embodiment 13, ethyl 1-cyclopropyl-6,7-difluoro-8-difluoromethoxyl-1,4-dihydro-4-oxoquinoline-3-carb oxylate (product compound of embodiment 3) is treated with [1R,6R]-2-tertbutyloxycarbonyl-2,8-diaza bicyclo[4,3,0] nonane (target product of embodiment 10) and then the amino-protecting group is removed with a solution of methanol/methyl acetate in anhydrous HCl to give the product, [ α ]²²D=+169.5°, (C=1).
¹H NMR(CF₃COOD) δ =1.08-1.49(4H, m), 1.90-2.00(4H, m), 2.91(1H, br.), 3.20(1H, br.), 3.57-3.60(1H, m), 3.97-4.41(6H, m), 6.48(1H, t, J=72.1Hz), 8.04(1H, d, J=13.5Hz), 9.24(1H, s). MS(m/z): 438(M⁺ +1).

### Embodiment 15

### Preparation of 1-Cyclopropyl-8-difluoromethoxyl-7-[cis-2,8-diazabicyclo[4,3,0]nonane-8-yl]-6-fl uoro-1,4-dihydro-4-oxoquinoline-3-carboxylic acid hydrochloride

As is in embodiment 13, ethyl 1-cyclopropyl-6,7-difluoro-8-difluoromethoxyl- ,4-dihydro-4-oxoquinoline-3-carb oxylic acid ester (target product of embodiment 3) is treated with cis-2-tertbutyloxycarbonyl-2,8-diaza bicyclo[4,3,0] nonane (target product of embodiment 11) to give the product.
¹H NMR(CF₃COOD) δ =1.10-1.52(4H, m), 1.91-2.01(4H, m), 2.92(1H, br.), 3.22(1H, br.), 3.58-3.62(1H, m), 3.98-4.41(6H, m), 6.48(1H, t, J=72.0Hz), 8.04(1H, d, J=13.5Hz), 9.24(1H, s). MS(m/z): 438(M⁺ +1).

### Embodiment 16

### Preparation of 1-Cyclopropyl-8-difluoromethoxyl-7-[cis-2,8-diazabicyclo[4,3,0]nonane-8-yl]-6-fl uoro-1,4-dihydro-4-oxoquinoline-3-carboxylic acid

Method 1 comprises neutralizing 1-cyclopropyl-8-difluoromethoxyl-7-[cis-2,8-diazabicyclo [4,3,0]nonane-8-yl]-6-fluoro-1,4-dihydro-4-oxoquinoline-3-carboxylic acid hydrochloride (target product of embodiment 15, 3.0 g) with ammonia water, refrigerating, filtering to obtain a solid, washing with cold water to give monohydrate of the product. MS(m/z): 438(M⁺ +1). Calculated result (measured value) of element analysis for the product: C 55.38(55.34), H 5.31 (5.45), N 9.23(9.25).

Method 2 comprises reacting boric acid (1.1 g,17.8 mmol) with acetic anhydride(6 mL,62.5 mmol) for 1.5 h at 110°C, slightly cooling, adding acetic acid (7.1 mL) to further react for 1 h at 110 °C , cooling to 50-60□ naturally, adding ethyl 1-cyclopropyl-6,7-difluoro-8-difluoro methoxyl-1,4-dihydro-4-oxoquinoline-3-carboxylate (target product of embodiment 3, 4.3 g, 12.0 mmol), stirring for 6 h, vacuum distilling to remove solvent, charging into ice water (150mL) under stirring, stirring for 0.5 h, filtering, washing obtained filter cake with water and ethanol in turn, drying to give a white solid (5.1 g), reacting the solid (0.5 g, 1.1 mmol) with cis-2-acetyl-2,8-diaza bicyclo[4,3,0]nonane (product compound of embodiment 12, 0.5 g, 2.9 mmol) in pyridine (20 mL) as solvent, stirring to react for 2 h at room temperature, distilling to remove solvent, adding 15% NaOH (10 mL) and ethanol (10 mL), refluxing for 15 h, distilling to remove ethanol, cooling down to room temperature, adjusting pH to 10-11 with 30% acetic acid, filtering, adjusting pH of obtained filtrate to 7.0 with 30% acetic acid, stirring for 0.5 h, refrigerating, filtering, and washing obtained solid with cold water to give the title compound (0.36 g, 75.0%). The properties of the compound are the same as the product of method 1.

### Embodiment 17

### Preparation of 5-amino-1-cyclopropyl-8-difluoromethoxyl-7-[(1S,6S)-2,8-diazabicyclo[4,3,0]non ane-8-yl]-6-fluoro-1,4-dihydro-4-oxoquinoline-3-carboxylic acid hydrochloride

The preparation comprises reacting 5-amino-1-cyclopropyl-6,7-difluoro-8-difluoromethoxyl-1,4-dihydro-4-oxoquinoline-3-carboxylic acid (compound of referential example 1, 0.50 g, 1.44 mmol) with [1S,6S]-2-tertbutyloxycarbonyl-2,8-diaza bicyclo[4,3,0]nonane (target product of embodiment 9, 0.90 g, 3.98 mmol) in pyridine (20 mL) as solvent for 0.5 h at 100°C, distilling to remove solvent and obtain residue (0.73 g), adding acetyl chloride (5 mL) slowly into methanol (10 mL) under ice-cooling, stirring for 30 min to prepare a solution of methanol/methyl acetate in anhydrous HCl, adding a solution of aforementioned residue (0.73 g) in methanol (5 mL), stirring at room temperature, distilling to remove solvent, adding ethylether (50 mL) to solidify the product, filtering, and subjecting obtained filter cake to recrystallization with ethanol to give 5-amino-l-cyclopropyl-8-ditluoromethoxyl-7-[(1S1S,6S)-2,8-diazabicyclo[4,3,0)non ane-8-yl]-6-fluoro-1,4-dihydro-4-oxoquinoline-3-carboxylic acid hydrochloride (0.45g). [ α ]²⁵D=-204.8° (DMSO,C=2).
¹H NMR(CDCl₃) δ =0.72-1.25(4H, m), 1.52-1.79(4H, m), 2.37(1H, br.), 2.68-2.71(1H, m), 3.05-3.09(1H, m), 3.38-3.44(3H, m), 3.97-4.03(3H, m), 6.21(1H, t, J=72.9Hz), 6.56(2H, br.), 8.65(1H,s). MS(m/z): 453(M⁺+1).

### Embodiment 18

### Preparation of 5-Amino-1-cyclopropyl-8-difluoromethoxyl-7-[(1R,6R)-2,8-diazabicyclo[4,3,0]no nane-8-yl]-6-fluoro-1,4-dihydro-4-oxoquinoline-3-carboxylic acid hydrochloride

As is in embodiment 17, 5-amino-1-cyclopropyl-6,7-difluoro-8-difluoromethoxyl-1,4-dihydro-4-oxoquinoli ne-3-carboxylic acid (referential example 1) compound is reacted with [1R,6R]-2-tertbutyloxycarbonyl-2,8-diaza bicyclo[4,3,0]nonane (embodiment 9 compound) to give the title compound, [ α ]²⁵D=+212.6° (DMSO,C=2).
¹H NMR(CDCl₃) δ =0.72-1.28(4H, m), 1.51-1.79(4H, m), 2.39(1H, br.), 2.68-2.73(1H, m), 3.06-3.11(1H, m), 3.38-3.42 (3H, m), 3.95-4.02(3H, m), 6.23(1H, t, J=72.9Hz), 6.57(2H, br.), 8.65(1H,s). MS(m/z): 453(M⁺ +1).

### Embodiment 19

### Preparation of 5-Amino-1-cyclopropyl-8-difluoromethoxyl-7-(cis-2,8-diazabicyclo[4,3,0]nonane-8-yl)-6-fluoro-1,4-dihydro-4-oxoquinoline-3-carboxylic acid trifluoroacetate

As is in embodiment 17, 5-amino-1-cyclopropyl-6,7-difluoro-8-difluoromethoxyl-1,4-dihydro-4-oxoquinoline-3-carboxylic acid (target product of referential example 1) is reacted with cis-2-tertbutyloxycarbonyl-2,8-diazabicyclo[4,3,0]nonane (compound of embodiment 11). The amino-protecting group is removed with trifluoroacetic acid, instead of the solution of methanol/methyl acetate in anhydrous HCl, to give the target compound. Calculated results (measured values) of element analysis for the product: C 48.77(48.84), H 4.27(4.19), and N 9.89(10.01).
¹H NMR(CDCl₃) δ =0.71-1.26(4H, m), 1.53-1.78(4H, m), 2.37(1H, br.), 2.69-2.72(1H, m), 3.04-3.09(1H, m), 3.38-3.46(3H, m), 3.99-4.06(3H, m), 6.20(1H, t, J=72.9Hz), 6.55(2H, br.), 8.64(1H, s). MS(m/z): 453(M⁺ +1).

The following salts can be likewise prepared, 5-amino-1-cyclopropyl-8-difluoromethoxyl-7-(cis-2,8-diazabicyclo[4,3,0]nonane-8-yl)-6-fluoro-1,4-dihydro-4-oxoquinoline-3-carboxylic acid acetate; 5-amino-1-cyclopropyl-8-difluoromethoxyl-7-(cis-2,8-diazabicyclo[4,3,0]nonane-8-yl)-6-fluoro-1,4-dihydro-4-oxoquinoline-3-carboxylic acid lactate; 5-amino-1-cyclopropyl-8-difluoromethoxyl-7-(cis-2,8-diazabicyclo[4,3,0]nonane-8-yl)-6-fluoro-1,4-dihydro-4-oxoquinoline-3-carboxylic acid maleate; 5-amino-1-cyclopropyl-8-difluoromethoxyl-7-(cis-2,8-diazabicyclo[4,3,0]nonane-8-yl)-6-fluoro-1,4-dihydro-4-oxoquinoline-3-carboxylic acid oxalate.

### Embodiment 20

### Preparation of 5-Amino-1-cyclopropyl-8-difluoromethoxyl-7-(cis-2,8-diazabicyclo[4,3,0]nonane-8-yl)-6-fluoro-1,4-dihydro-4-oxoquinoline-3-carboxylic acid mesylate

As is in embodiment 17, 5-amino-1-cyclopropyl-6,7-difluoro-8-difluoromethoxyl-1,4-dihydro-4-oxoquinoli ne-3-carboxylic acid (target product of referential example 1) is reacted with cis-2-tertbutyloxycarbonyl-2,8-diazabicyclo[4,3,0]nonane (target product of embodiment 11); then the amino-protecting group is removed by reacting with a solution of methylsulfonic acid in methanol, instead of the solution of methanol/methyl acetate in anhydrous HCl, to give the title compound. Calculated results (measured values) of element analysis for the product: C 48.17(48.20), H 4.96(5.12), and N 10.21(10.18).

The following salts can be likewise prepared,

5-amino-1-cyclopropyl-8-difluoromethoxyl-7-(cis-2,8-diazabicyclo[4,3,0]nonane-8-yl)-6-fluoro-1,4-dihydro-4-oxoquinoline-3-carboxylic acid p-toluene sulfonate.

### Embodiment 21

### Preparation of 5-Amino-1-cyclopropyl-8-difluoromethoxyl-7-(cis-2,8-diazabicyclo[4,3,0]nonane-8-yl)-6-fluoro-1,4-dihydro-4-oxoquinoline-3-carboxylic acid

The preparation comprises neutralizing 5-amino-1-cyclopropyl-8-difluoromethoxyl-7-(cis-2,8-diazabicyclo[4,3,0]nonane-8-yl)-6-fluoro-1,4-dihydro-4-oxoquinoline-3-carboxylic acid trifluoroacetate (target product of embodiment 19, 3.0 g) with ammonia water, refrigerating, filtering, and washing obtained solid with cold water to give monohydrate of the title compound. MS(m/z):453(M⁺ +1). Calculated results (measured values) of element analysis for the product: C 53.62(53.58), H 5.36(5.48), and N 11.91(11.87).

### Embodiment 22

### The preparation of coated tablet

Core ingredients

| | |
|---|---|
| Target compound of embodiment 13 | 10.0 g |
| Lactose | 50.0 g |
| Starch | 40.0 g |
| Hydroxypropyl cellulose | 4.0 g |
| 10% Polyvinylpyrrolidone in ethanol | qs |
| Magnesium stearate | 0.5 g |

The aforementioned ingredients are mixed uniformly, pelletized, sieved, dried, and then tableted into 100 core pieces.

Coating liquid ingredients
Opadry 5 g in 80% ethanol

### Embodiment 23

### Preparation of capsule

Ingredients

| | |
|---|---|
| Target compound of embodiment 14 | 100 g |
| Starch | 10 g |
| Sodium carboxymethylcellulose | 20 g |
| Low-substituted hydroxypropyl cellulose | 10 g |
| Tween 80 | qs |
| 5% Polyvinylpyrrolidone in ethanol | qs |
| Sodium laurylsulfate | 8 g |
| 0# capsule dissolvable in stomach | 1,000 pieces |

The above ingredients are made into 1,000 pieces of capsules.

### Preparation method

The method comprises respectively sieving prescription amount of materials, adding 5% polyvinylpyrrolidone alcohol solution and Tween 80, sieving with 20 mesh sieve, air drying at room temperature around 15°C, mixing with sodium laurylsulfate, loading into 0# capsule dissolvable in stomach in the amount of 0.27 g/S, sampling, and assaying, wherein the dissolution Limit Q=80%, and the content should be 90-110% of the labeled value.

### Embodiment 24

### Preparation of injection

The preparation comprises dissolving target compound of embodiment 17 1 g in appropriate amount of water for injection, adding poloxamer 10 g, sodium chloride 4 g, dextran 10 g, glucose 4 g and mannitol 5 g, mixing, adding water for injection to 1,000 mL, and preparing into 10 vials of intravenous injection.

## Claims

1. Quinolonecarboxylic acid derivatives of formula (I), pharmaceutically acceptable salts or hydrates thereof, 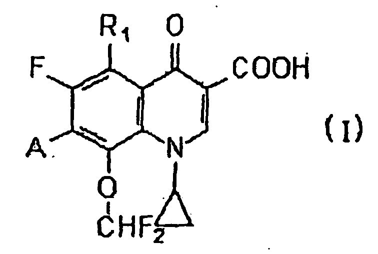 wherein R₁ is a hydrogen atom or an amino group, A has a structure of formula (II) with (1S, 6S) configuration, a structure of formula (III) with (1R, 6R) configuration, or a structure of formula (IV) with cis-racemic configuration, 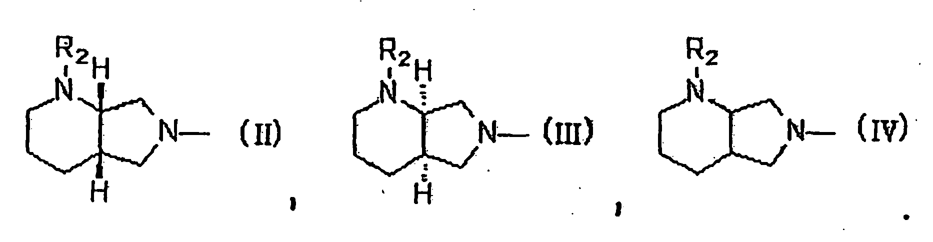 in which R₂ is a hydrogen atom or an amino-protecting group, wherein the amino-protecting group is formyl, acetyl, trifluoroacetyl, benzoyl, paranitrobenzoyl, p-toluene sulfonyl, methoxycarbonyl, ethoxycarbonyl, fluorenylmethoxycarbonyl, t-butyl-oxycarbonyl (BOC) or trichloroethoxycarbonyl.

2. The quinolonecarboxylic acid denvatives,pharmaceutically acceptable salts or hydrates thereof according to claim 1, wherein the salts are ones of inorganic

3. The quinolonecarboxylic acid derivatives, pharmaceutically acceptable salts or hydrates thereof according to claim 2, wherein the salts of inorganic acids are a salt of hydrochloric acid, hydrobromic acid, phosphoric acid or sulfuric acid.

4. The quinolonecarboxylic acid derivatives, pharmaceutically acceptable salts or hydrates thereof according to claim 2, wherein the salts of organic acids are a salt of methanesulfonic acid, paratoluenesulfonic acid, acetic acid, trifluoroacetic acid, lactic acid, citric acid, maleic acid, oxalic acid, succinic acid, benzoic acid, tartaric acid, fumaric acid, mandelic acid, ascorbic acid or malic acid.

5. A method for preparing the quinolonecarboxylic acid derivatives, pharmaceutically acceptable salts or hydrates thereof according to claims 1-4, wherein said method comprises reacting compound of formula (V) with compound of formula (VI), then removing the amino-protecting group of compound (I), and treating with an acid to give corresponding salt of compound (I), optionally, the salt of compound (I) is further dissolved in water or other aqueous organic solvent to form a solution, and pH of the solution is adjusted to 6-8 with an alkaline aqueous solution or an alkaline aqueous organic solvent, wherein after the solution cools down, the hydrate of compound (I) is separated out, wherein R₁ is a hydrogen atom or an amino group, R₃ is an amino-protecting group, and X is a halogen atom

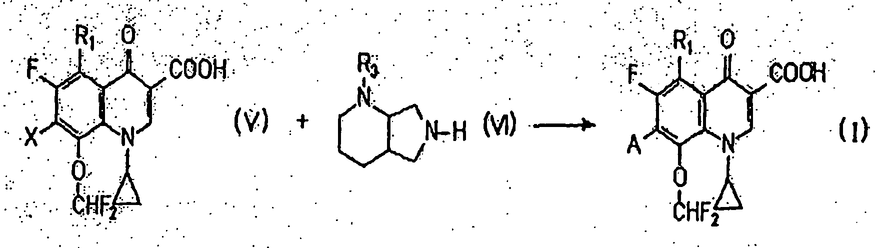

6. The method according to claim 5 comprises reacting compound of formula (V) with compound of formula (VI) in the presence of acid-binding agent at temperature in the range from room temperature to 200°C under stirring for 1-20 h, and removing amino-protecting group of compound (I),
wherein the amino-protecting group is formyl, acetyl, trifluoroacetyl, benzoyl, paranitrobenzoyl, p-toluene sulfonyl, methoxycarbonyl, ethoxycarbonyl, fluorenyl methoxycarbonyl, t-butyl-oxycarbonyl (BOC) or trichloroethoxycarbonyl, and the halogen atom is fluorine, chlorine or bromine.

7. A method for preparing the quinolonecarboxylic acid derivatives, pharmaceutically acceptable salts or hydrates thereof according to claims 1-4, wherein said method comprises firstly converting compound (VII) into compound (VIII), and then reacting compound (VIII) with compound (VI) to obtain compound (I) via intermediate compound (IX), wherein the use of a proper acid in the N-deprotection of the compound (I) yields a corresponding salt of the compound (I), optionally, the salts of compound (I) are further dissolved in water or other water-containing organic solvent to form a solution, to which an alkaline aqueous solution or alkaline aqueous organic solvent is added to adjust pH to within 6-8, and cooled to separate out hydrate of the compound (I), 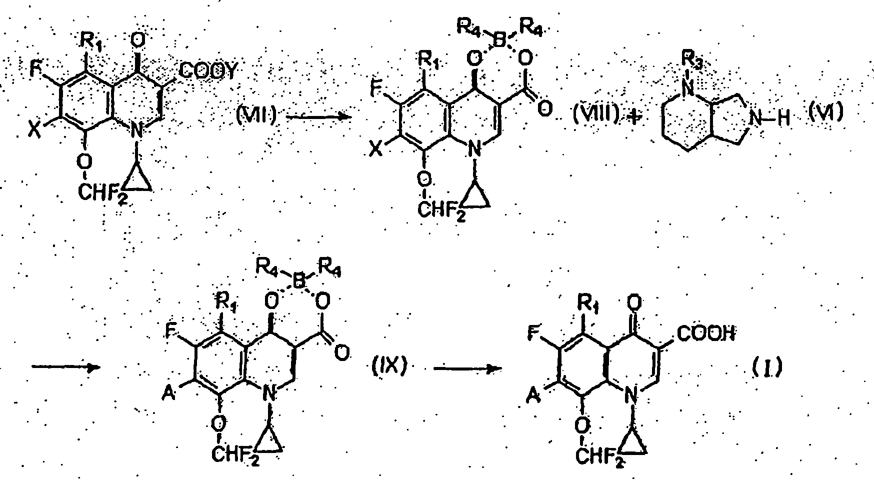 wherein R₁ is a hydrogen atom or an amino group, Y is hydrogen atom, C₁-C₆ alkyl group, benzyl, and R₄ is a C₂-C₆ aliphatic acid group, C₂-C₆ halogenated aliphatic acid group or C₇-C₁₁ aromatic acid group, and
Formula (VI) concerning the chiral compound represents optical isomer with (1S,6S), (1R,6R) configuration or cis-racemic configuration.

8. The method according to claim 7 comprises reacting compound (VIII) with compound (VI) in the presence of acid-binding agent under stirring for 1-20 h at temperature in the range from room temperature to 200°C to give compound (IX), and removing boron-containing group and amino-protecting group of the compound (IX).

9. Drug compositions containing quinolonecarboxylic acid derivatives pharmaceutically acceptable salts or hydrates thereof according to claims 1-4, wherein the drug compositions contain physiologically effective quantity of quinolonecarboxylic acid derivatives 0.1-99.9 wt % and/or pharmaceutically acceptable carrier 0.1-99.9 wt%.

10. The drug compositions according to claim 9, wherein the drug compositions are in form of pharmaceutically appropriate preparation.

11. The drug compositions according to claim 10, wherein the preparation can be tablet, sugar-coated tablet, film-coated tablet, enteric coating tablet, slow release tablet, capsule, hard capsule, soft capsule, slow release capsule, oral liquid, mistura, buccal tablet, granule, pill, pulvis, concentrated extract, bolus, suspensoid, liquor, injection, powder and injection preparation, freeze drying powder and injection preparation, powder, dispersible powder, cachet, suppositorium, ointment, plaster, cream, spray, aerosol, gutt, or patch.

12. The drug compositions according to claim 10, wherein when the drug compositions are solid or semisolid pharmaceutical preparation in the form of powder, tablet, dispersible powder, capsule, cachet, suppositorium or ointment, solid carrier can be used.

13. The drug compositions according to claim 12, wherein the solid carrier used is selected from diluent, flavoring agent, solubilizing agent, lubricant, suspending agent, binder, and swelling agent, or it can be coating material.

14. The drug compositions according to claim 13, wherein the solid carrier is selected from one or more of magnesium carbonate, magnesium stearate, talcum, sucrose, lactose, pectin, dextrin, starch, gelatin, tragacanth, methyl cellulose, sodium carboxymethylcellulose, low boiling point wax, and cocoa butter.

15. Pharmaceutical uses of the quinolonecarboxylic acid derivatives, pharmaceutically acceptable salts or hydrates thereof according to claims 1-4 for preparing antibacterial agents.

## Patentansprüche

1. Chinoloncarbonsäurederivate der Formel (I), pharmazeutisch annehmbare Salze oder Hydrate davon, 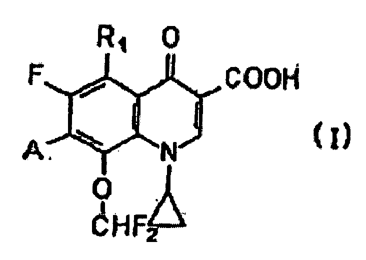 worin R₁ ein Wasserstoffatom oder eine Aminogruppe ist, A eine Struktur der Formel (II) mit (1 S, 6S)-Konfiguration, eine Struktur der Formel (III) mit (1 R, 6R)-Konfiguration oder eine Struktur der Formel (IV) mit razemischer cis-Konfiguration hat, 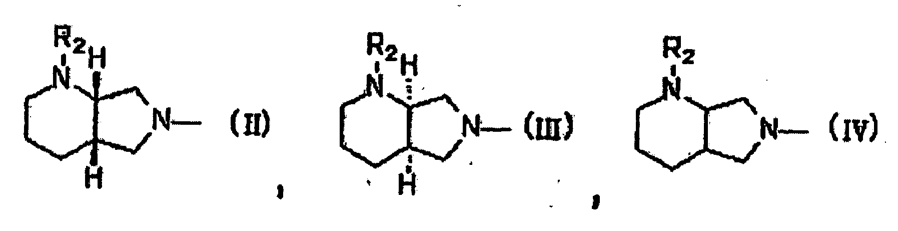 worin R₂ ein Wasserstoffatom oder eine Amino-Schutzgruppe ist, wobei
die Aminoschutzgruppe Formyl, Acetyl, Trifluoracetyl, Benzoyl, Paranitrobenzoyl, p-Toluolsulfonyl, Methoxycarbonyl, Ethoxycarbonyl, Fluorenylmethoxycarbonyl, t-Butyloxycarbonyl (BOC) oder Trichlorethoxycarbonyl ist.

2. Chinoloncarbonsäurederivate, pharmazeutisch annehmbare Salze oder Hydrate davon nach Anspruch 1, wobei die Salze von anorganischen Säuren oder organischen Säuren stammen.

3. Chinoloncarbonsäurederivate, pharmazeutisch annehmbare Salze oder Hydrate davon nach Anspruch 2, wobei es sich bei den Salzen von anorganischen Säuren um ein Salz der Chlorwasserstoffsäure, Bromwasserstoffsäure, Phosphorsäure oder Schwefelsäure handelt.

4. Chinoloncarbonsäurederivate, pharmazeutisch annehmbare Salze oder Hydrate davon nach Anspruch 2, wobei es sich bei den Salzen von organischen Säuren um ein Salz der Methansulfonsäure, Paratoluolsulfonsäure, Essigsäure, Trifluoressigsäure, Milchsäure, Zitronensäure, Maleinsäure, Oxalsäure, Bernsteinsäure, Benzoesäure, Weinsäure, Fumarsäure, Mandelsäure, Ascorbinsäure oder Apfelsäure handelt.

5. Verfahren zur Herstellung der Chinoloncarbonsäurederivate, pharmazeutisch annehmbaren Salze oder Hydrate davon nach Anspruch 1-4, wobei das Verfahren umfasst: Reagieren der Verbindung der Formel (V) mit einer Verbindung der Formel (VI), dann Entfernen der Aminoschutzgruppe der Verbindung (I) und Behandeln mit einer Säure, um ein entsprechendes Salz der Verbindung (I) zu ergeben, wahlweise wird das Salz der Verbindung (I) weiter in Wasser oder einem anderen wässrigen organischen Lösungsmittel gelöst, um eine Lösung zu bilden, und der pH-Wert der Lösung wird mit einer alkalischen wässrigen Lösung oder einem alkalischen wässrigen organischen Lösungsmittel auf 6-8 eingestellt, wobei nach dem Abkühlen der Lösung das Hydrat der Verbindung (I) abgetrennt wird, wobei R₁ ein Wasserstoffatom oder eine Aminogruppe ist, R₃ eine Aminoschutzgruppe ist und X ein Halogenatom darstellt,

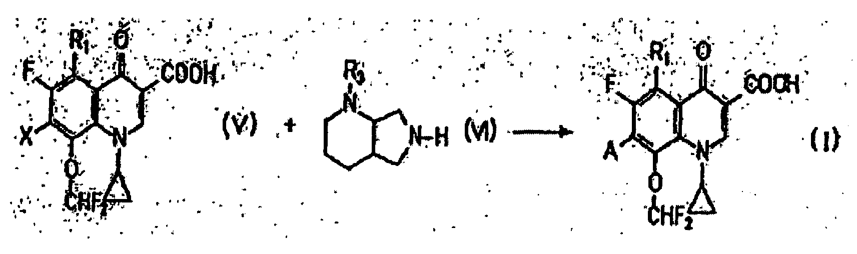

6. Verfahren nach Anspruch 5, umfassend das Reagieren einer Verbindung der Formel (V) mit einer Verbindung der Formel (VI) in Gegenwart eines Säurebindemittels bei einer Temperatur im Bereich von Raumtemperatur bis 200°C unter Rühren für 1-20 h und das Entfernen der Aminoschutzgruppe der Verbindung (I),
wobei die Aminoschutzgruppe Formyl, Acetyl, Trifluoracetyl, Benzoyl, Paranitrobenzoyl, p-Toluolsulfonyl, Methoxycarbonyl, Ethoxycarbonyl, Fluorenylmethoxycarbonyl, t-Butyloxycarbonyl (BOC) oder Trichlorethoxycarbonyl ist und das Halogenatom Fluor, Chlor oder Brom ist.

7. Verfahren zur Herstellung der Chinoloncarbonsäurederivate, pharmazeutisch annehmbaren Salze oder Hydrate davon nach Anspruch 1-4, wobei das Verfahren umfasst: zunächst Umwandeln der Verbindung (VII) in die Verbindung (VIII) und dann Reagieren der Verbindung (VIII) mit der Verbindung (VI) zum Erzielen der Verbindung (I) über die Zwischenverbindung (IX), wobei die Verwendung einer geeigneten Säure beim Entfernen der N-Schutzgruppe der Verbindung (I) ein entsprechendes Salz der Verbindung (I) ergibt, wahlweise die Salze der Verbindung (I) weiter in Wasser oder einem anderen, Wasser enthaltenden organischen Lösungsmittel gelöst werden, um eine Lösung zu bilden, zu der eine alkalische wässrige Lösung oder ein alkalisches wässrigen organisches Lösungsmittel gegeben wird, um den pH-Wert auf innerhalb 6-8 einzustellen, und gekühlt werden, um das Hydrat der Verbindung (I) abzutrennen, 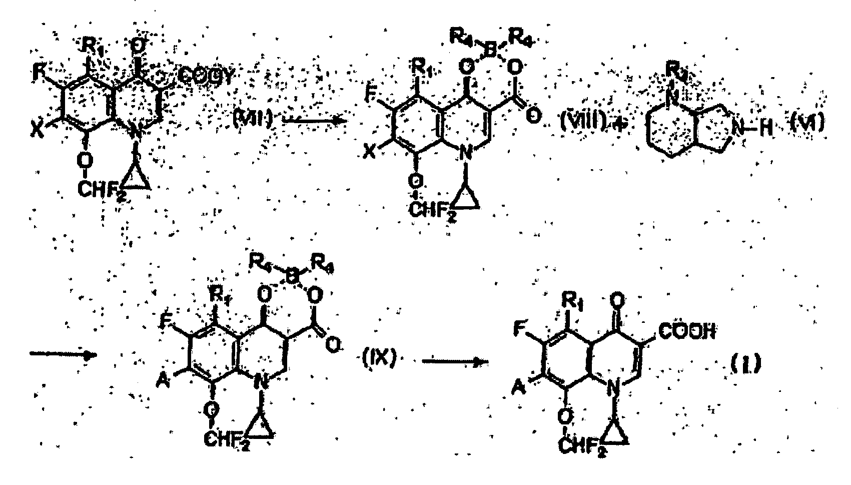 wobei R₁ ein Wasserstoffatom oder eine Aminogruppe ist, Y ein Wasserstoffatom, eine C₁-C₆-Alkylgruppe, Benzyl ist und R₄ eine aliphatische C₂-C₆-Säuregruppe, halogenierte aliphatische C₂-C₆-Säuregruppe oder aromatische C₇-C₁₁-Säuregruppe ist, und
Formel (VI) betreffend die chirale Verbindung ein optisches Isomer mit (1S,6S),(1R,6R)-Konfiguration oder razemischer cis-Konfiguration darstellt.

8. Verfahren nach Anspruch 7, umfassend das Reagieren der Verbindung (VIII) mit der Verbindung (VI) in Gegenwart eines Säurebindemittels unter Rühren für 1-20 h bei einer Temperatur im Bereich von Raumtemperatur bis 200°C, um eine Verbindung (IX) zu ergeben, und das Entfernen der Bor enthaltenden Gruppe und Aminoschutzgruppe der Verbindung (IX).

9. Medikamentenzusammensetzungen, enthaltend Chinoloncarbonsäurederivate, pharmazeutisch annehmbare Salze oder Hydrate davon nach Anspruch 1-4, wobei die Medikamentenzusammensetzungen eine physiologisch wirksame Menge an Chinoloncarbonsäurederivate von 0,1 - 99,9 Gew.-% und/oder einen pharmazeutisch annehmbaren Träger von 0,1 - 99,9 Gew.-% enthalten.

10. Medikamentenzusammensetzungen nach Anspruch 9, wobei die Medikamentenzusammensetzungen in Form eines pharmazeutisch geeigneten Präparats vorliegen.

11. Medikamentenzusammensetzungen nach Anspruch 10, wobei das Präparat eine Tablette, mit Zucker überzogene Tablette, Filmtablette, Tablette mit magensaftresistentem Überzug, Tablette mit langsamer Freisetzung, Kapsel, Hartkapsel, Weichkapsel, Kapsel mit langsamer Freisetzung, orale Flüssigkeit, Mixtura, bukkale Tablette, Granulat, Pille, Pulvis, konzentrierten Extrakt, Bolus, Suspensoid, Liquor, Injektion, Pulver- und Injektionspräparat, gefriergetrocknetes Pulver- und Injektionspräparat, Pulver, dispergierbares Pulver, Päckchen, Suppositorium, Salbe, Verband, Creme, Spray, Aerosol, Tropfen oder Pflaster sein kann.

12. Medikamentenzusammensetzungen nach Anspruch 10, wobei der feste Träger verwendet werden kann, wenn es sich bei den Medikamentenzusammensetzungen um ein festes oder halbfestes pharmazeutisches Präparat in Form eines Pulvers, einer Tablette, eines dispergierbaren Pulvers, einer Kapsel, eines Päckchens, Suppositoriums oder einer Salbe handelt.

13. Medikamentenzusammensetzungen nach Anspruch 12, wobei der verwendete feste Träger aus Verdünnungsmitteln, Aromastoffen, Lösungsvermittlern, Schmiermitteln, Suspensionsmitteln, Bindemitteln und Quellmitteln ausgewählt ist oder ein Überzugsmaterial sein kann.

14. Medikamentenzusammensetzungen nach Anspruch 13, wobei der feste Träger aus einem oder mehreren der folgenden ausgewählt ist:
Magnesiumcarbonat, Magnesiumstearat, Talk, Saccharose, Lactose, Pektin, Dextrin, Stärke, Gelatine, Tragant, Methylcellulose,
Natriumcarboxymethylcellulose, Wachs mit niedrigem Siedepunkt und Kakaobutter ausgewählt ist.

15. Pharmazeutische Verwendungen von Chinoloncarbonsäurederivaten, pharmazeutisch annehmbaren Salzen oder Hydraten davon nach Anspruch 1-4 zur Herstellung von antibakteriellen Mitteln.

## Revendications

1. Dérivés d'acide carboxylique de quinolone selon la formule (I), sels ou hydrates pharmaceutiquement acceptables de ceux-ci, 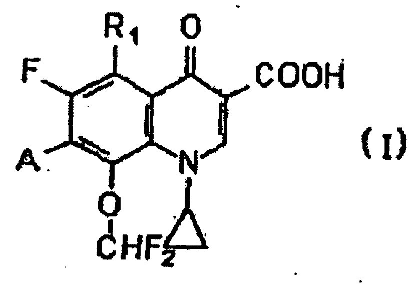 dans lesquels R₁ est un atome d'hydrogène ou un groupe amino, A possède une structure selon la formule (II) avec une configuration (1S, 6S), une structure selon la formule (III) avec une configuration (1R, 6R) ou une structure selon la formule (IV) avec une configuration cis-racémique, 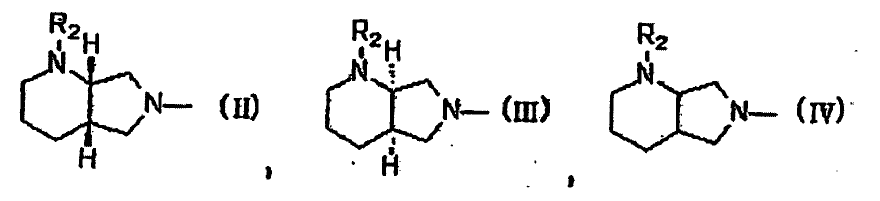 dans lesquels R₂ est un atome d'hydrogène ou un groupe amino-protecteur, pour lesquels ce groupe amino-protecteur est un formyle, un acétyle, un trifluoroacétyle, un benzoyle, un paranitrobenzoyle, un sulfonyle de toluène-p, un méthoxycarbonyle, un éthoxycarbonyle, un méthoxycarbonyle de fluorényle, un t-butyle-oxycarbonyle (BOC) ou un trichloroéthoxycarbonyle.

2. Dérivés d'acide carboxylique de quinolone, sels ou hydrates pharmaceutiquement acceptables de ceux-ci selon la revendication 1, pour lesquels les sels sont des sels d'acides inorganiques ou d'acides organiques.

3. Dérivés d'acide carboxylique de quinolone, sels ou hydrates pharmaceutiquement acceptables de ceux-ci selon la revendication 2, pour lesquels les sels d'acides inorganiques sont des sels d'acide chlorhydrique, d'acide bromhydrique, d'acide phosphorique ou d'acide sulfurique.

4. Dérivés d'acide carboxylique de quinolone, sels ou hydrates pharmaceutiquement acceptables de ceux-ci selon la revendication 2, pour lesquels les sels d'acides organiques sont des sels d'acide méthanesulfonique, d'acide paratoluènesulfonique, d'acide acétique, d'acide trifluoroacétique, d'acide lactique, d'acide citrique, d'acide maléique, d'acide oxalique, d'acide succinique, d'acide benzoïque, d'acide tartarique, d'acide fumarique, d'acide mandélique, d'acide ascorbique ou d'acide malique.

5. Procédé de préparation des dérivés d'acide carboxylique de quinolone, de sels ou d'hydrates pharmaceutiquement acceptables de ceux-ci selon les revendications 1 à 4, pour lequel ledit procédé comprend les étapes consistant à faire réagir un composé selon la formule (V) avec un composé selon la formule (VI), puis retirer le groupe amino-protecteur du composé (I) et traiter avec un acide pour donner un sel correspondant du composé (I), de façon optionnelle le sel du composé (I) est en outre dissous dans de l'eau ou un autre solvant organique aqueux pour former une solution, et le pH de cette solution est ajusté à 6 à 8 avec une solution aqueuse alcaline ou un solvant organique aqueux alcalin, pour lequel, après que la solution se soit refroidie, l'hydrate du composé (I) est séparé, pour lequel R₁ est un atome d'hydrogène ou un groupe amino, R₃ est un groupe amino-protecteur et X est un atome d'halogène.

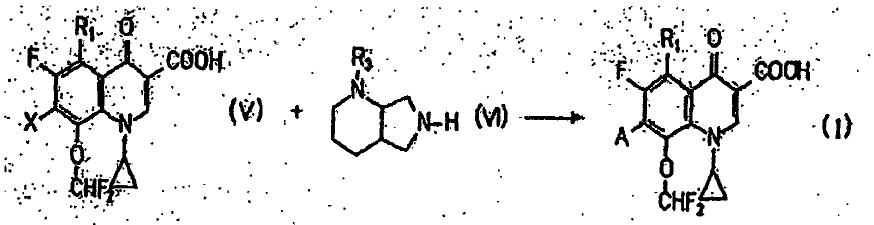

6. Procédé selon la revendication 5, comprenant les étapes consistant à faire réagir un composé selon la formule (V) avec un composé selon la formule (VI) en présence d'un agent de fixation aux acides à une température dans la gamme allant d'une température ambiante jusqu'à 200 °C sous agitation pendant 1 à 20 heures, puis retirer le groupe amino-protecteur du composé (I),
pour lequel le groupe amino-protecteur est un formyle, un acétyle, un trifluoroacétyle, un benzoyle, un paranitrobenzoyle, un sulfonyle de toluène-p, un méthoxycarbonyle, un éthoxycarbonyle, un méthoxycarbonyle de fluorényle, un t-butyle-oxycarbonyle (BOC) ou un trichloroéthoxycarbonyle et l'atome d'halogène est un atome de fluor, de chlore ou de brome.

7. Procédé de préparation des dérivés d'acide carboxylique de quinolone, de sels ou d'hydrates pharmaceutiquement acceptables de ceux-ci selon les revendications 1 à 4, pour lequel ledit procédé comprend les étapes consistant d'abord à convertir le composé (VII) en un composé (VIII), puis à faire réagir le composé (VIII) avec le composé (VI) pour obtenir le composé (I), en passant par le composé intermédiaire (IX), pour lequel l'utilisation d'un acide approprié pour la dé-protection N du composé (I) produit un sel correspondant du composé (I), de façon optionnelle les sels du composé (I) sont en outre dissous dans de l'eau ou un autre solvant organique contenant de l'eau pour former une solution, à laquelle une solution aqueuse alcaline ou un solvant organique aqueux alcalin est ajouté pour ajuster le pH dans une gamme de 6 à 8, et refroidis pour séparer l'hydrate du composé (I), 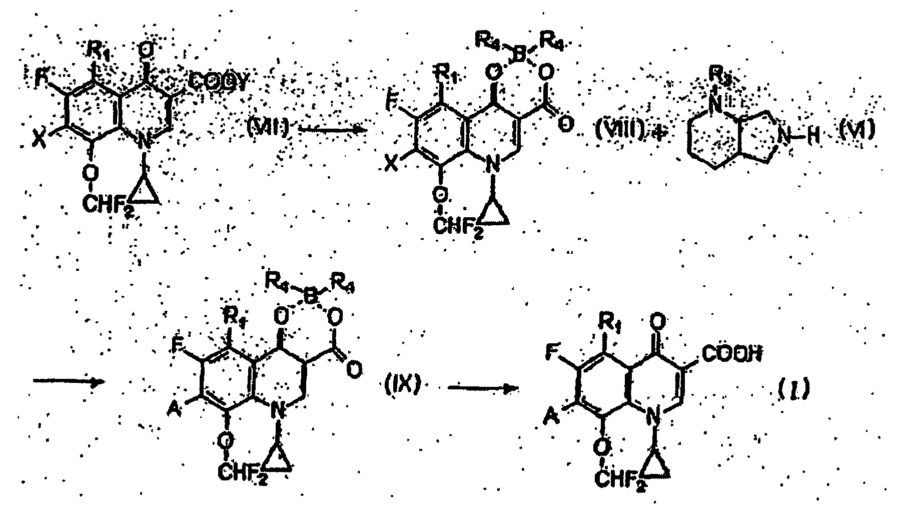 pour lequel R₁ est un atome d'hydrogène ou un groupe amino, Y est un atome d'hydrogène, un groupe alkyle C₁ à C₆ ou un benzyle et R₄ est un groupe d'acide aliphatique C₂ à C₆, un groupe d'acide aliphatique halogéné C₂ à C₆ ou un groupe d'acide aromatique C₇ à C₁₁, et
la formule (VI) concernant le composé chiral représente un isomère optique avec une configuration (1S, 6S), une configuration (1R, 6R) ou une configuration cis-racémique.

8. Procédé selon la revendication 7, comprenant les étapes consistant à faire réagir un composé (VIII) avec un composé (VI) en présence d'un agent de fixation aux acides, sous agitation pendant 1 à 20 heures à une température dans la gamme allant d'une température ambiante jusqu'à 200 °C pour donner un composé (IX), et retirer un groupe contenant du bore et un groupe amino-protecteur du composé (IX).

9. Compositions de médicaments contenant des dérivés d'acide carboxylique de quinolone, des sels ou hydrates pharmaceutiquement acceptables de ceux-ci selon les revendications 1 à 4, pour lesquelles les compositions de médicaments contiennent une quantité physiologiquement efficace de dérivés d'acide carboxylique de quinolone de 0,1 à 99,9 % en masse et/ou un vecteur pharmaceutiquement acceptable à raison de 0,1 à 99,9 % en masse.

10. Compositions de médicaments selon la revendication 9, pour lesquelles les compositions de médicaments ont la forme de préparations pharmaceutiquement appropriées.

11. Compositions de médicaments selon la revendication 10, pour lesquelles les préparations peuvent être des comprimés, des comprimés dragéifiés, des comprimés recouverts d'un film, des comprimés à couverture entérique, des comprimés à libération lente, des gélules, des gélules dures, des gélules souples, des gélules à libération lente, des liquides pour voie orale, des mixtures, des comprimés buccaux, des granulés, des pilules, des poudres, des extraits concentrés, des bolus, des suspensoïdes, des liqueurs, des injections, des préparations de poudres à injecter, des préparations de poudres lyophilisées à injecter, des poudres, des poudres dispersibles, des cachets, des suppositoires, des pommades, des plâtres, des crèmes, des liquides vaporisés, des aérosols, des gouttes ou des patch.

12. Compositions de médicaments selon la revendication 10, pour lesquelles, lorsque les compositions de médicaments sont des préparations pharmaceutiques solides ou semi-solides, sous la forme de poudres, de comprimés, de poudres dispersibles, de gélules, de cachets, de suppositoires ou de pommades, un vecteur solide peut être utilisé.

13. Compositions de médicaments selon la revendication 12, pour lesquelles le vecteur solide utilisé est sélectionné parmi un diluant, un agent aromatique, un agent solubilisant, un lubrifiant, un agent de suspension, un liant et un agent d'adsorption ou il peut être une substance d'enrobage.

14. Compositions de médicaments selon la revendication 13, pour lesquelles le vecteur solide est sélectionné à raison d'une ou plusieurs substances parmi un carbonate de magnésium, un stéarate de magnésium, un talc, une saccharose, un lactose, une pectine, une dextrine, un amidon, une gélatine, une gomme adragante, une méthyle-cellulose, une sodium carboxyméthyle-cellulose, une cire à point d'ébullition bas et un beurre de cacao.

15. Utilisation pharmaceutique des dérivés d'acide carboxylique de quinolone, des sels ou hydrates pharmaceutiquement acceptables de ceux-ci selon les revendications 1 à 4, pour la préparation d'agents antibactériens.
